# FASCICULE DE BREVET EUROPEEN

(11) **EP 1 837 073 B1**
(45) Date de publication et mention de la délivrance du brevet: **06.01.2016**
(21) Numéro de dépôt: 07300881.5
(22) Date de dépôt: 20.03.2007
(51) Int. Cl.: A61Q 1/06, B01J 13/16, A61K 8/87, A61K 8/02

(54) **Composition cosmétique et/ou dermatologique pour le maquillage et/ou le soin d'une matière kératinique et procédé de traitement cosmétique des matières kératiniques comprenant l'application sur lesdites matières de la composition cosmétique**
Kosmetische und / oder dermatologische Zusammensetzung, insbesondere zum Schminken und / oder zur Pflege einer Keratinsubstanz und Verfahren zur kosmetischen Behandlung von Keratinsubstanzen mit der kosmetische Zusammensetzung
Cosmetic and/or dermatological composition for making up and/or caring for a keratin material and cosmetic process for treating keratin materials with the composition

(30) Priorité: 24.03.2006 FR 0651031
(43) Date de publication de la demande: 26.09.2007
(73) Titulaire: L'Oréal, 75008 Paris (FR)
(72) Inventeur: JEANNE-ROSE, Valérie, 95100 Argenteuil (FR); Rodriguez, Yvan, 60290, CAUFFRY (FR)
(74) Mandataire: Le Coupanec, Pascale A.M.P.

(56) Documents cités:
- WO-A-03/101606
- WO-A-2006/013165
- WO-A2-2005/102251
- JP-B2- 3 301 082
- US-A- 5 155 165
- US-A- 5 911 923
- US-B2- 6 890 653

## Description

La présente invention concerne une composition cosmétique et/ou dermatologique, caractérisée en ce qu'elle comprend au moins un matériau colorant composite se présentant sous la forme de microcapsules composées d'une matrice polymérique et comprenant un pigment.

D'une manière générale, les compositions cosmétiques visent à procurer un effet esthétique, en association ou non avec un soin, au niveau du site d'application auquel elles sont dédiées. Cet effet esthétique est, dans la plupart des cas, figuré par un effet coloré procuré par des substances colorantes présentes dans la composition cosmétique.

A titre illustratif de compositions dédiées plus particulièrement à la génération d'un effet coloré, on peut par exemple citer les rouges à lèvres, les fonds de teint, les fards à paupières, les fards à joues, les crayons à yeux, ou encore les mascaras.

Bien entendu, les substances colorantes utilisables pour la fabrication de compositions cosmétiques et/ou dermatologiques destinées au maquillage et/ou au soin d'une matière kératinique doivent répondre à des normes imposées en terme d'innocuité. Ainsi, il existe pour un certain nombre de pays des listes répertoriant en particulier les pigments dont l'usage à des fins de maquillage est autorisé.

Ces listes sont contraignantes à plusieurs titres. D'une part, en n'autorisant qu'un nombre limité de pigments, elles ne permettent pas d'accéder à une large palette colorielle. D'autre part, ces listes diffèrent selon les pays considérés et ne possèdent donc en commun qu'un nombre réduit de pigments. Par conséquent, le nombre de pigments utilisables sur un large espace économique pour le maquillage d'une matière kératinique est relativement restreint.

Il existe donc un besoin d'élargir la gamme colorimétrique accessible à partir d'un pigment donné, et notamment à partir d'un pigment agréé pour une zone économique importante.

Par ailleurs, il est connu que certains pigments présentent une aptitude au dégorgement. Ce dégorgement peut entraîner de la griffe, par exemple sur les lèvres (notamment dans le cas des rouges à lèvres) ou sur les ongles (notamment dans le cas des vernis à ongles), et se traduit donc par une altération de la qualité du maquillage résultant. Les techniques d'enrobage classiques (basées sur les silicones ou les perfluoroalkyles, par exemple) ne permettent malheureusement pas de limiter le dégorgement observé.

Il existe donc également un besoin de limiter le dégorgement des pigments dans l'eau.

La présente invention vise précisément à donner satisfaction sur ces deux aspects.

Les inventeurs ont découvert, de façon surprenante, que l'encapsulation d'un pigment par polycondensation-réticulation interfaciale permet d'obtenir une poudre organique dans laquelle est emprisonné ledit pigment permettant, d'une part, d'accéder à des propriétés colorimétriques nouvelles par rapport au même pigment sous forme libre et, d'autre part, de s'affranchir de tout problème de dégorgement.

Les techniques de microencapsulation sont fréquemment employées dans l'industrie phytosanitaire ou encore dans celle du papier autocopieur.

Ainsi, on connaît du document GB 1 091 141 un procédé d'encapsulation de liquides par polymérisation interfaciale, conduisant notamment à la formation de microcapsules formées d'une matrice polymérique réticulée de type polyamide, polyester, polyurée, polycarbonate ou polyamide-polyurée.

Le brevet US 6 753 083 décrit, pour sa part, des compositions utiles pour encapsuler divers matériaux, et en particulier des catalyseurs, dans une matrice polymérique réticulée ou non, et pouvant être obtenue par polymérisation interfaciale.

Enfin, il est également connu, d'après les brevets US 6 890 653 et WO 03/101606, d'encapsuler des substances organiques hydrosolubles, comme par exemple des phéromones, dans des microcapsules formées d'une matrice polymérique de type polyuréthane et/ou polyurée, en vue notamment de contrôler leur libération.

Toutefois, aucun de ces documents n'applique spécifiquement une telle technique à l'encapsulation d'un pigment dans le domaine des cosmétiques et notamment à des fins d'élargir la gamme colorielle accessible à partir d'un unique pigment.

Le document WO2005/102251 A2 décrit une composition comportant, dans un milieu physiologiquement acceptable, au moins un pigment composite comportant un noyau inorganique et au moins un enrobage au moins partiel d'au moins une matière colorante organique.

Ainsi, la présente invention décrit un matériau colorant composite caractérisé en ce qu'il se présente sous la forme de microcapsules composées d'une matrice polymérique de type polyuréthane, polyurée et/ou polyuréthane/polyurée réticulée et obtenue par polycondensation interfaciale, et d'un pigment.

Selon un premier de ses aspects, la présente invention concerne une composition cosmétique et/ou dermatologique pour le maquillage et/ou le soin d'une matière kératinique, caractérisée en ce qu'elle comprend un milieu physiologiquement acceptable, ainsi que :
au moins un matériau colorant composite se présentant sous la forme de microcapsules composées :
   (a) d'une matrice polymérique de type polyuréthane, polyurée et/ou polyuréthane et polyurée réticulée obtenue par polycondensation interfaciale en faisant réagir
      au moins un diisocyanate choisi parmi les mélanges de monomères diphénylméthane diisocyanates et d'oligomères diphénylméthane diisocyanates, le 2,4-tolylène diisocyanate et le 2,6-tolylène diisocyanate, ainsi que leur mélange, le 4,4'-diphénylméthane diisocyanate ou l'isophorone diisocyanate ;
      avec au moins un réactif de type alcool et/ou amine portant au moins trois fonctions, identiques ou différentes, choisies parmi les fonctions hydroxyle et amine, en présence éventuellement d'au moins un diol et/ou d'au moins une diamine et/ou d'au moins un amino-alcool difonctionnel, et
   (b) d'un pigment, lesdites microcapsules comprenant de 10 à 80 % en poids du pigment par rapport au poids de la matrice polymérique.

Selon un autre de ses aspects, la présente invention concerne un procédé de traitement cosmétique des matières kératiniques, notamment de la peau du corps ou du visage, des ongles, des cheveux et/ou des cils, comprenant l'application sur lesdites matières d'une composition cosmétique telle que définie précédemment.

L'invention décrit encore l'utilisation d'au moins un matériau colorant composite selon l'invention pour la préparation d'une composition cosmétique pour le maquillage et/ou le soin d'une matière kératinique.

Avantageusement, les matériaux colorants composites possèdent une couleur différente de celle du pigment d'origine qu'ils contiennent, c'est-à-dire différente de celle du pigment sous forme non encapsulée.

En outre, ces matériaux composites se présentant sous la forme d'une poudre, ils peuvent être mis en oeuvre aisément dans tous types de compositions cosmétiques, et sous une large gamme de conditions opératoires. Les matériaux peuvent en particulier être formulés dans des produits cosmétiques nécessitant au cours de leur fabrication une étape de chauffage à haute température, tels par exemple les rouges à lèvres. Ils se distinguent ainsi avantageusement des pigments en solution ou en dispersion dans un solvant volatil, ou susceptible de se volatiliser durant le procédé de fabrication, ou encore dans un solvant incompatible avec les autres composés.

Les inventeurs ont également découvert que l'encapsulation d'un pigment par polycondensation-réticulation interfaciale permet de limiter le dégorgement dans l'eau dudit pigment.

Dans le cadre de la présente invention, le terme « matière kératinique » désigne la peau, les lèvres, les ongles, les cheveux, les cils et les sourcils et le terme « fibres kératiniques » désigne plus particulièrement les cheveux, les cils et les sourcils.

Les compositions selon l'invention comprennent un milieu physiologiquement acceptable, notamment cosmétiquement acceptable, c'est-à-dire un milieu compatible en particulier avec les matières kératiniques, et notamment les fibres kératiniques telles les cheveux, les cils et les sourcils.

Par « cosmétiquement acceptable », on entend, dans le cadre de la présente invention, un composé dont l'utilisation est compatible avec une application sur les matières et les fibres kératiniques.

Au sens de l'invention, les microcapsules sont des particules de forme sphérique constituées d'un matériau de support, appelé encore « matrice polymérique » dans la présente description, *via* lequel est emprisonné un pigment.

### Matrice polymérique

Les matrices polymériques convenant à la mise en oeuvre de la présente invention sont de type polyuréthane, polyurée et/ou polyuréthane et polyurée et sont réticulées.

Selon un mode de réalisation préféré de l'invention, la matrice polymérique des microsphères selon l'invention est de type polyuréthane et polyurée.

La matrice polymérique décrite peut être obtenue en faisant réagir au moins deux réactifs, l'un dit de type "isocyanate" et l'autre dit de type "alcool et/ou amine", l'un au moins de ces deux réactifs portant au moins trois fonctions, identiques ou différentes, choisies pour le premier type parmi les fonctions isocyanate, et pour le second type parmi les fonctions hydroxyle et amine. Ce réactif assure notamment la fonction de réticulant.

Une matrice polymérique décrite peut par exemple être obtenue en faisant réagir un réactif de type alcool et/ou amine comportant au moins trois fonctions, identiques ou différentes, choisies parmi les fonctions hydroxyle et amine, avec un réactif de type isocyanate comportant une seule, voire de préférence deux, fonction(s) isocyanate.

Une matrice polymérique décrite peut également être obtenue en faisant réagir un tri- ou un poly-isocyanate avec un réactif de type alcool et/ou amine comportant une seule, voire de préférence deux, fonction(s), identiques ou différentes le cas échéant, choisies parmi les fonctions hydroxyle et amine.

L'eau peut également agir comme réactif et générer un groupe amino, par addition sur un groupe NCO et élimination consécutive du CO₂, ledit groupe amino pouvant ensuite réagir *in situ* avec un groupe NCO.

### Réactifs de type isocyanate

Des réactifs de type isocyanate peuvent généralement comprendre une seule ou plusieurs, et notamment deux, voire trois fonctions isocyanate. Des di- ou poly-isocyanates aliphatiques, cycloaliphatiques, araliphatiques, aromatiques et hétérocycliques sont décrits par exemple par W. SIEFKEN dans Justus Liebigs Annalen der Chemie, 562, pages 75 à 136.

Les réactifs de type isocyanate selon l'invention sont choisis parmi les diisocyanates. Sont connus parmi les diisocyanates l'éthylène diisocyanate, le 1,4-tétraméthylène diisocyanate, le 1,6-hexaméthylène diisocyanate, le 2,2,4- ou 2,4,4-triméthyl-1,6-hexaméthylène diisocyanate, le 1,12-dodécane diisocyanate, le cyclobutane 1,3-diisocyanate, le cyclohexane 1,3- et 1,4-diisocyanate et tout mélange de ses isomères, le 4,4'-méthylènebis(cyclohéxyl)diisocyanate le 1-isocyanato-3,3,5-triméthyl-5-isocyanatométhylcyclohexane, le 2,4- et 2,6-hexane-hydrotolylène diisocyanate et tout mélange de ses isomères, l'hexahydro-1,3- et 1,4-phénylène diisocyanate, le perhydro-1,4'-et 4,4'-diphénylméthane diisocyanate, le 1,3- et 1,4-phénylène diisocyanate, le 2,6-tolylène diisocyanate et tout mélange de ses isomères, le 4,4'-diphénylméthanediisocyanate (4,4'-MDI), le 1,5-napthylène diisocyanate, le m-xylylène diisocyanate, le tétraméthylxylylène diisocyanate, les m- et p-isocyanatophénylsulfonyl isocyanates, le lysine alkyl ester diisocyanate où l'alkyl est en C₁ à C₁₀ ou le 2-butyl-2-éthylpentaméthylène diisocyanate, et leurs mélanges.

Des réactifs de type isocyanate connus peuvent comprendre au moins trois fonctions isocyanate et jouer ainsi le rôle de réticulant. Ils peuvent alors notamment être choisis parmi les triisocyanates, comme par exemple le triphénylméthane 4,4',4"-triisocyanate, ou le 4-isocyanatométhyl-1,8-octaneméthylène diisocyanate ou encore parmi les polyisocyanates, et notamment les polyphénylpolyméthylène polyisocyanates, les aryl poly-isocyanates perchlorés, les polyisocyanates ayant des groupes carbodiimides, les polyisocyanates ayant des groupes allophanates, les polyisocyanates ayant des groupes isocyanurates, les poly-isocyanates ayant des groupes urée acylés, les polyisocyanates ayant des groupes bis-urée, les poly-isocyanates préparés par réaction de télomérisation, les polyisocyanates ayant des groupes éthers, les produits de réaction des isocyanates mentionnés ci-dessus avec des acétals, les poly-isocyanates contenant des radicaux d'acides gras polymériques et tout mélange des poly-isocyanates mentionnés ci-dessus.

Il existe également, à titre de réactif de type isocyanate, des mélanges desdits isocyanates, c'est-à-dire des mélanges d'isocyanates aliphatiques, des mélanges des isocyanates aromatiques, des mélanges d'isocyanates aliphatiques et aromatiques, et en particulier des mélanges qui comprennent éventuellement des diphénylméthane diisocyanates modifiés.

A titre illustratif de ces mélanges, on peut notamment citer le biuréthique hexaméthylène diisocyanate, en mélange avec le 4,4'-diphénylméthane isocyanate, et éventuellement avec le 2,4-diphénylméthane isocyanate, l'hexaméthylène diisocyanate trimérisé, en mélange avec le 4,4'-diphénylméthane diisocyanate, et éventuellement avec le 2,4-diphénylméthane diisocyanate.

Il existe également, à titre de réactif de type isocyanate, des oligo- ou poly-isocyanates susceptibles d'être préparés à partir des di- ou poly-isocyanates cités précédemment ou leurs mélanges en les liant au moyen de structures uréthane, allophanate, urée, bis-urée, amide, isocyanurate, carbodiimide, uretonimine, oxadiazinetrione ou iminooxadiazinedio ne.

On peut encore citer les di- ou poly-isocyanates, tels que les mélanges de monomères diphénylméthane diisocyanates et d'oligomères diphénylméthane diisocyanates (également appelés polymères MDI), le 2,4-tolylène diisocyanate (2,4-TDI), le 2,4'-diphénylméthane diisocyanate (2,4'-MDI), le triisocyanatotoluène, l'isophorone diisocyanate (IPDI), le 2-butyl-2-ethylpentaméthylène diisocyanate, le 2-isocyanatopropylcyclohexyl isocyanate, le 3(4)-isocyantométhyl-1-méthylcyclohexyl isocyanate, le 1,4-diisocyanato-4-méthylpentane, le 2,4'-méthylènebis(cyclohexyl) diisocyanate et le 4-méthylcylcohexane 1,3-diisocyanate (H-TDI), qui peuvent plus particulièrement être utilisés à titre de réactif de type isocyanate.

Ainsi, selon l'invention, les réactifs de type isocyanate utilisables pour former les matériaux selon l'invention sont choisis parmi les mélanges de monomères diphénylméthane diisocyanates et d'oligomères diphénylméthane diisocyanates (polymères MDI), le 2,4-tolylène diisocyanate et le 2,6-tolylène diisocyanate, ainsi que leur mélange, le 4,4'-diphénylméthane diisocyanate (4,4'-MDI) ou encore l'isophorone diisocyanate (IPDI).

La quantité de réactif de type isocyanate à utiliser pour la mise en oeuvre de l'invention varie dans la gamme utilisée de façon usuelle dans les procédés de polyaddition interfaciale, et dépend en particulier du taux de microencapsulation recherché.

### Réactif de type alcool et/ou amine

Les réactifs de type alcool et/ou amine utilisables pour former les matériaux selon l'invention comprennent au moins trois fonctions, identiques ou différentes, choisies parmi les fonctions hydroxyle et amine.

Ils peuvent par exemple être choisis parmi les réactifs de type alcool, les réactifs de type amine, et les réactifs de type aminoalcools, utilisés seuls ou en mélange les uns avec les autres.

Ils ont de préférence un poids moléculaire allant de 200 à 4000 g/mol.

### Réactifs de type alcool

Les réactifs de type alcool utilisables pour former les matériaux selon l'invention peuvent comprennent au moins trois fonctions hydroxyle.

Plus particulièrement, ces réactifs peuvent être un polyol.

Par polyol, on entend au sens de l'invention toute molécule organique comportant dans sa structure chimique au moins deux groupements hydroxyle -OH.

Un polyol est généralement un composé hydrocarboné, linéaire, ramifié ou cyclique, saturé ou insaturé portant au moins deux fonctions hydroxyle.

Un polyol peut être en particulier un composé (hydro)carboné comportant de préférence de 2 à 300 atomes de carbone, et portant au moins deux groupes hydroxyle, de préférence de 2 à 10 groupes hydroxyle.

De préférence, il s'agit d'un composé (hydro)carboné ayant de 3 à 32 atomes de carbone, et notamment de 4 à 18 atomes de carbone, voire de 4 à 12 atomes de carbone.

En particulier, un polyol peut être un composé ayant de 2 à 18 atomes de carbone et de 2 à 6 fonctions hydroxyles.

Des réactifs de type alcool connus sont les diols et notamment les dérivés du glycol tels que le diéthylène glycol, le dipropylène glycol, l'éthylène glycol, le propylène glycol, l'hexylène glycol, l'isoprène glycol, le butylène glycol, et le pentylène glycol, ou encore le butane diol, le propane-1,2 diol, les pentanediols et en particulier le 1,5-pentanediol, le décanediol, le dodécanediol, ou leurs mélanges.

Selon la présente invention, les réactifs de type alcool comprennent au moins trois fonctions hydroxyle, et jouent ainsi le rôle de réticulant. Ils peuvent alors notamment être choisis parmi le triméthylolpropane, le glycérol, le pentaérythritol, le 1,2,3-trihydroxyhexane, l'érythritol, l'arabitol, l'adonitol, le dulcitol et le sorbitol, les polymères et les copolymères de glycérol, comme par exemple l'hexaglycérol et le diglycérol, les dérivés du glycérol, comme par exemple le butyldiglycol, le polyglycéryl-3-diisostéarate et l'huile de ricin, les dérivés du glycol comme par exemple les polyéthylène glycols et notamment les polyéthylène glycols (PEG) ayant de 4 à 150 motifs d'éthylène glycol, comme le PEG-400, le PEG-600, le PEG-800, et le PEG-1200, les polypropylène glycols, les copolymères de l'éthylène glycol et du propylène glycol ou encore les sucres tels que le glucose, le fructose, le xylose, le tréhalose, le saccharose, le maltose, le lactose, et leurs mélanges.

De manière préférée, les réactifs de type alcool utiles pour jouer le rôle de réticulant sont choisis parmi le triméthylolpropane, le glycérol, le pentaérythritol et les sucres.

Un polyol peut également être un polyéther alcool de poids moléculaire moyen allant de 150 à 600, tel que le polyéthylène glycol 300 et la polyglycérine 500.

Il existe aussi des mélanges des polyols mentionnés ci-dessus.

Un polyol peut être également un polyol non éthérifié ou un polyol non estérifié.

Des réactifs de type alcool existant sont des diols, comme par exemple le diéthylène glycol, des polyols, comme par exemple les polyéthylènes glycols, et notamment ceux ayant de 4 à 150 motifs d'éthylène glycol, ou encore des mélanges de polyéthylène glycols et de diéthylène glycol.

### Réactifs de type amine

Les réactifs de type amine comprennent généralement une seule ou plusieurs, et notamment deux, voire trois fonctions amine.

Il existe des diamines, comme par exemple le diaminoéthane, les diaminopropanes, les diaminobutanes, les diaminohexanes, la pipérazine, la 2,5-diméthyl-pipérazine, l'amino-3-aminométhyl-3,5,5-triméthylcyclohexane (isophorone diamine, IPDA), le 4,4'-diaminodicyclohexylméthane, le 1,4-diaminocyclohexane, l'aminoéthyléthanolamine, l'hydrazine ou l'hydrate d'hydrazine.

Selon la présente invention, les réactifs de type amine comprennent au moins trois fonctions amine, et jouent ainsi le rôle de réticulant. Ils peuvent alors notamment être choisis parmi les triamines, comme par exemple la guanidine, la diéthylène triamine ou le 1,8-diamino-4-aminométhyloctane.

Les réactifs de type amine peuvent également être utilisés sous forme de cétimines, cétazines ou des sels d'amines correspondants.

Selon un mode de réalisation préféré, les réactifs de type amine utilisables pour former les matériaux selon l'invention comprennent au moins un groupe amino choisi parmi les groupes amines primaires et amines secondaires de type NHR, dans lequel R représente un groupe alkyle ayant de 1 à 8 atomes de carbone.

Les réactifs de type amine sont de préférence solubles dans l'eau en tant que tels, ou sous la forme d'un de leurs sels.

### Réactifs de type aminoalcool

Les réactifs de type aminoalcool utilisables pour former les matériaux selon l'invention peuvent comprennent au moins trois fonctions différentes choisies parmi les fonctions amine et hydroxyle.

Il existe des réactifs de type aminoalcools difonctionnels, c'est-à-dire qui comprennent deux fonctions, à savoir une fonction amine et une fonction hydroxyle.

Selon la présente invention, les réactifs de type aminoalcool comprennent au moins trois fonctions, et jouent ainsi le rôle de réticulant. Ils peuvent par exemple comprendre une unique fonction hydroxyle (respectivement amine) et au moins deux fonctions amine (respectivement hydroxyle), ou encore deux fonctions hydroxyle (respectivement amine) et au moins une fonction amine (respectivement hydroxyle).

Comme réactif de type aminoalcool utilisable dans la présente invention, on peut notamment citer l'éthanolamine et la triéthanolamine.

Comme précisé précédemment, les matrices polymériques des microcapsules selon l'invention sont obtenues en faisant réagir au moins un diisocyanate tel que défini précédemment, avec au moins un réactif de type alcool et/ou amine portant au moins trois fonctions, identiques ou différentes, choisies parmi les fonctions hydroxyle et amine, et jouant le rôle de réticulant, en présence éventuellement d'au moins un diol et/ou d'au moins une diamine et/ou d'au moins un amino-alcool difonctionnel tels que décrits ci-dessus.

En particulier, le réactif de type isocyanate est un diisocyanate choisi parmi les mélanges de monomères diphénylméthane diisocyanates et d'oligomères diphénylméthane diisocyanates (polymères MDI), le 2,4-tolylène diisocyanate et le 2,6-tolylène diisocyanate, ainsi que leur mélange, le 4,4'-diphénylméthane diisocyanate (4,4'-MDI) ou encore l'isophorone diisocyanate (IPDI).

Selon un mode de réalisation préféré, le réactif jouant le rôle de réticulant est choisi parmi les triols, comme par exemple le triméthylolpropane, le glycérol et le pentaérythritol, et les polyols, comme par exemple les sucres tels le glucose, le fructose, le xylose, le tréhalose, le saccharose, le maltose et le lactose.

Sont décrits à titre de diamines celles comprenant au moins un groupe amino choisi parmi les groupes amines primaires et amines secondaires de type -NHR, dans lequel R représente un groupe alkyle ayant de 1 à 8 atomes de carbone.

Les matrices polymériques selon l'invention sont notamment obtenues par polycondensation interfaciale d'au moins deux réactifs tels que définis ci-dessus.

La polycondensation interfaciale est une réaction de polymérisation qui se produit à l'interface de deux liquides non miscibles, dont au moins l'un des deux contient un réactif polyfonctionnel approprié.

Selon un mode de réalisation préféré, au moins un monomère et/ou réactif est soluble dans la première phase et au moins un monomère et/ou réactif est soluble dans la deuxième phase, non miscible à ladite première phase.

Dans le cas où cette réaction s'effectue sans élimination de produits secondaires, elle est également appelée polymérisation interfaciale.

Cette réaction peut être notamment réalisée dans une émulsion, notamment biphasique voire multiphasique, à l'interface entre les phases non miscibles.

Dans le cadre de la présente demande, la réaction peut être réalisée dans une émulsion de type huile-dans-eau ou eau-dans-huile, ou encore dans une émulsion multiple de type eau-dans-huile-dans-eau ou huile-dans-eau-dans huile.

Selon un mode de réalisation préféré, l'émulsion est du type huile-dans-eau.

Comme précisé précédemment, les matrices polymériques convenant à la mise en oeuvre de la présente invention sont réticulées.

Cette réticulation est obtenue par réaction des chaînes de polymères avec un réactif de fonctionnalité au moins égale à 3, appelé réticulant, dont des exemples sont donnés ci-dessus.

Par suite de la réticulation de la matrice polymérique, les effets induits sur le pigment encapsulé selon l'invention, et par exemple la modulation de la teinte, sont irréversibles.

Suivant la structure de la matrice polymérique, on distingue deux types de microcapsules :
- les microcapsules de type matriciel, appelées également microsphères, dans lesquelles la matrice polymérique est un réseau continu, dans lequel est dispersée le pigment à encapsuler,
- les microcapsules de type réservoir dans lesquelles la matrice polymérique est une enveloppe solide, encore appelée « écorce », d'épaisseur variable, délimitant un coeur dans lequel est emprisonnée le pigment à encapsuler.

Dans le cadre de la présente invention, les microcapsules de type réservoir peuvent emprisonner ledit pigment dans leur écorce et/ou dans leur coeur.

Lorsque la réaction est réalisée dans une émulsion, et si les oligomères formés au début de la réaction sont insolubles dans la phase dispersée, la formation de microcapsules de type réservoir est privilégiée. En revanche, si lesdits oligomères sont solubles dans la phase dispersée, la formation de microcapsules de type matriciel est alors privilégiée.

Lorsque la polymérisation est réalisée en l'absence de solvant, les microcapsules obtenues sont de type matriciel.

Lorsque la polymérisation est réalisée en l'absence de solvant mais en présence d'une huile, notamment cosmétique, ne solvatant pas les oligomères formés, les microcapsules obtenues sont de type réservoir.

Selon un mode de réalisation de l'invention, les microcapsules sont de type réservoir.

Selon un autre mode de réalisation, le pigment selon l'invention est emprisonné dans l'écorce des microcapsules de type réservoir.

Selon un autre mode de réalisation, le pigment selon l'invention est emprisonné dans le coeur des microcapsules de type réservoir.

Le pigment selon l'invention peut également être emprisonné à la fois dans l'écorce et dans le coeur des microcapsules de type réservoir.

Selon un autre mode de réalisation de l'invention, les microcapsules sont de type matriciel.

Au sens de la présente invention, et sans précision complémentaire, le terme « microcapsule » entend aussi bien couvrir les microcapsules de type matriciel que les microcapsules de type réservoir.

Les microcapsules selon l'invention ont une taille comprise entre 500 nm et 1200 µm, notamment entre 500 nm et 30 µm.

La taille des microcapsules peut par exemple être mesurée par microscopie optique, électronique, ou encore par granulo-laser.

### Pigments

Par pigment on entend désigner une particule solide, blanche ou colorée, naturellement insoluble dans les phases hydrophiles et lipophiles liquides usuellement employées en cosmétique ou rendue insoluble par formulation sous forme de laque, le cas échéant.

Un pigment peut par exemple désigner une particule solide, blanche ou colorée, insoluble dans une solution d'isododécane lorsqu'elle y est présente en une teneur de 10 % en poids, par rapport au poids total de ladite solution, à 25 °C et à 1 atm.

Par « insoluble » s'entend, au sens de l'invention, qu'il reste dans la phase liquide, et en particulier dans la solution d'isododécane, des particules en suspension ou que le mélange n'est pas limpide, après mélange et agitation pendant 30 minutes.

Dans le cadre de la présente invention, le pigment peut être au moins en partie organique.

Selon un mode de réalisation de l'invention, le pigment est un pigment organique.

Selon un autre mode de réalisation de l'invention, le pigment est un pigment minéral.

Les microcapsules selon l'invention comprennent de 10 à 80 % en poids, par exemple de 15 à 75 % en poids, notamment de 20 à 70 % en poids, voire de 25 à 65 % en poids et de préférence de 30 à 60 % en poids du pigment par rapport au poids de la matrice polymérique.

Par « par rapport au poids de la matrice polymérique », s'entend que la teneur correspondante se rapporte au poids de la matrice polymérique sans tenir compte du pigment.

En d'autres termes, cela signifie que la teneur correspondante se rapporte au poids en monomère(s) entrant dans la composition de la matrice polymérique.

Bien entendu, le taux d'encapsulation dépend de la modulation de la teinte recherchée et peut donc varier significativement selon l'effet que l'on souhaite obtenir.

A titre illustratif des pigments utilisables dans la présente invention, on peut citer le noir de carbone, l'oxyde de titane, l'oxyde de chrome, les pigments de type D&C, FD&C et leurs laques, et notamment ceux connus sous les dénominations D&C Blue n°4, D&C Brown n°1, FD&C Green n°3, D&C Green n°5, D&C Green n°6, FD&C Green n°8, D&C Orange n°4, D&C Orange n°5, D&C Orange n°10, D&C Orange n°11, FD&C Red n°4, D&C Red n°6, D&C Red n°7, D&C Red n°17, D&C Red n°21, D&C Red n°22, D&C Red n°27, D&C Red n°28, D&C Red n°30, D&C Red n°31, D&C Red n°33, D&C Red n°34, D&C Red n°36, FD&C Red n°40, FD&C Red 40 lake, D&C Violet n°2, Ext. D&C Violet n°2, FD & C Blue n° 1, D&C Yellow n°6, FD&C Yellow n°6, D&C Yellow n°7, Ext. D&C Yellow n°7, D&C Yellow n°8, D&C Yellow n°10 ou D&C Yellow n°11, étant entendu que lorsque ledit pigment n'est pas naturellement insoluble dans les phases hydrophiles et lipophiles usuellement employées en cosmétique, il est utilisé sous forme d'une laque correspondante.

A titre d'exemple de laques, on peut notamment citer les laques à base de baryum, strontium, calcium, aluminium, ou encore les dicéto pyrrolopyrrole.

Comme autre exemple de pigments utilisables dans la présente invention, on peut notamment citer les pigments minéraux, éventuellement traités en surface et/ou enrobés, et notamment le dioxyde de titane, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, ou encore les poudres métalliques comme la poudre d'aluminium, la poudre de cuivre, la poudre d'or et la poudre d'argent.

On peut également citer les pigments à effet optique tels que les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, ou l'oxyde de chrome.

Il peut également s'agir de nacres.

Par nacres, il faut comprendre des pigments irisés, notamment produits par certains mollusques dans leur coquille ou bien synthétisés.

Les pigments nacrés peuvent être choisis parmi le mica recouvert de titane ou d'oxychlorure de bismuth, le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser des pigments interférentiels, notamment à cristaux liquides ou multicouches.

Il peut également s'agir de pigments ayant une structure qui peut être par exemple de type séricite/oxyde de fer brun/dioxyde de titane/silice.

Il peut également s'agir de pigments ayant une structure qui peut être par exemple de type microsphères de silice contenant de l'oxyde de fer.

A titre d'exemples de pigments et de laques convenant tout particulièrement à la mise en oeuvre de la présente invention, on peut notamment citer le D&C Red n°7, l'oxyde de titane, l'oxyde de chrome, les laques des pigments de type D&C et FD&C cités ci-dessus, et notamment le D&C Red n°22 lake, le Yellow n°6 lake, le FD&C Blue n°1 lake.

### Autres substances non colorantes

Les matériaux colorants composites selon l'invention peuvent également comprendre, en outre, au moins une substance non colorante qui peut être choisie parmi les huiles et les cires cosmétiques, notamment minérales, animales, végétales ou de synthèse, les actifs cosmétiques tels que les vitamines, les filtres UV, les parfums et les hydratants.

La substance non colorante additionnelle peut être emprisonnée dans l'écorce ou dans le coeur de la microcapsule, lorsque celle-ci est de type réservoir, indépendamment de la localisation du pigment.

La substance non colorante additionelle peut être présente dans les microcapsules selon l'invention en une teneur allant de 0,001 à 60 % en poids par rapport au poids de la matrice polymérique.

En particulier, les huiles cosmétiques additionnelles peuvent être présentes dans les microcapsules selon l'invention en une teneur allant de 10 à 60 % en poids par rapport au poids total de la matrice polymérique.

Les actifs cosmétiques additionnels peuvent être présents en une teneur allant de 0,001 à 10 % en poids par rapport au poids total de la matrice polymérique.

La taille des microcapsules peut être avantageusement contrôlée par adjonction d'une quantité minimale d'une huile hydrophobe, de préférence hydrocarbonée, telle que par exemple l'isododécane ou le parléam, et/ou d'esters d'acide gras, notamment de benzoate d'alcool gras, en une teneur allant notamment de 5 à 20 % en poids, par rapport au poids total de la matrice polymérique.

Les matériaux colorants composites selon l'invention peuvent être obtenus par polycondensation interfaciale, et plus particulièrement par le procédé de préparation suivant.

Ce procédé peut être réalisé à une température variant de 0 °C à 100 °C. L'émulsification de la phase aqueuse et de la phase organique est obtenue par agitation, par exemple en utilisant un homogénéisateur de type Ultraturrax^{®} ou sonicateur (sonde à ultrasons) à une température variant de 2 °C à 40 °C, et préférentiellement de l'ordre de 15 °C.

Après émulsification, les comonomères hydrosolubles sont introduits dans la phase aqueuse et la température du milieu réactionnel est élevée jusqu'à une température comprise variant de 60 °C à 100 °C, et préférentiellement de l'ordre de 65 °C, afin d'initier la polymérisation.

Selon l'invention, la polymérisation peut être menée en présence ou en l'absence d'un catalyseur, tel que par exemple le 2-éthylhexanoate d'étain.

En fin de polymérisation, la poudre peut être récupérée par centrifugation (4000 tr/min pendant 30 minutes) ou par filtration, ou éventuellement par lyophilisation. La poudre ainsi obtenue est ensuite lavée à l'eau et à l'éthanol, puis séchée à l'air.

La phase aqueuse est généralement constituée d'eau, mais il peut également s'agir d'une solution aqueuse contenant un solvant organique hydrosoluble.

La phase aqueuse peut, en outre, contenir des stabilisants, des colloïdes tels que par exemple l'alcool polyvinylique (PVA) ou la polyvinylpyrolidone (PVP), et des tensioactifs ioniques et par exemple le lauryl sulfate de sodium (SDS), ou non ioniques comme par exemple les copolymères blocs polyéthyleneoxyde-polypropylèneoxyde-polyéthyleneoxyde de la gamme PLURONIC.

Elle peut également contenir le(s) monomère(s) hydrosoluble(s).

La phase organique contient le(s) monomère(s) hydrophobe(s) (réactifs de type isocyanate) et le pigment.

Elle peut contenir un solvant des monomères isocyanates tels que le toluène, le xylène, ou une huile volatile (isododécane) ou non volatile minérale (parléam, benzoate d'alcool), ou végétale, ou animale, ou siliconée (diméthylsiloxane, silicone phénylée) ou une cire ou un mélange de ces huiles et cires. La phase organique peut également contenir un agent de dispersion du pigment (Solsperse^{®}).

Les matériaux colorants composites peuvent être utilisés dans les domaines cosmétiques et/ou dermatologiques notamment pour colorer et/ou opacifier une composition cosmétique et/ou dermatologique.

Ainsi, l'invention concerne, selon un de ses aspects, une composition cosmétique et/ou dermatologique, en particulier pour le maquillage et/ou le soin d'une matière kératinique, caractérisée en ce qu'elle comprend au moins un matériau colorant composite.

Une composition selon l'invention peut comprendre de 0,01 % à 50 % en poids, notamment de 0,1 à 20 % en poids, et en particulier de 0,5 à 15 % en poids, par rapport à son poids total, de matériau colorant composite tel que défini ci-dessus.

Les compositions cosmétiques ou dermatologiques selon l'invention comprennent, outre ledit matériau colorant composite, un milieu physiologiquement acceptable, notamment cosmétiquement ou pharmaceutiquement acceptable, c'est-à-dire un milieu compatible avec les matières kératiniques telles que la peau du visage ou du corps, les lèvres, les cheveux, les cils, les sourcils et les ongles.

Les compositions selon l'invention peuvent ainsi comprendre, selon l'application envisagée, les constituants habituels à ce type de composition.

Ainsi, les compositions selon l'invention peuvent comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et/ou les polyols comme la glycérine, la diglycérine, le propylène glycol, le sorbitol, le pentylène glycol, et les polyéthylène glycols; et/ou des éthers en C₂ et des aldéhydes en C₂-C₄ hydrophiles; et/ou des esters à chaîne courte (ayant de 3 à 8 atomes de carbone au total) tels que l'acétate d'éthyle, l'acétate de méthyle, l'acétate de propyle, l'acétate de n-butyle, ou l'acétate d'isopentyle.

Les compositions selon l'invention peuvent avantageusement comprendre une phase grasse liquide, qui peut elle-même comprendre des huiles et/ou des solvants de préférence lipophiles; des cires, des gommes et/ou des corps gras pâteux, d'origine végétale, animale, minérale ou de synthèse, voire siliconés, et leurs mélanges.

Comme cire susceptible d'être présente dans une composition selon l'invention, on peut par exemple citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25 °C. Il est également possible d'utiliser des cires de silicone, comme par exemple les alkyls, alcoxys et/ou esters de polyméthylsiloxane.

Les compositions selon l'invention peuvent également comprendre des huiles d'origine minérale, animale, végétale ou synthétique, carbonées, hydrocarbonées, fluorées et/ou siliconées, volatiles ou non volatiles, seules ou en mélange dans la mesure où elles forment un mélange homogène et stable et où elles sont compatibles avec l'utilisation envisagée.

Comme huile susceptible d'être présente dans une composition selon l'invention, on peut notamment citer, seules ou en mélange, les huiles hydrocarbonées telles que l'huile de paraffine ou de vaseline; le perhydrosqualène; l'huile d'arara; l'huile d'amande douce, de calophyllum, de palme, de ricin, d'avocat, de jojoba, d'olive ou de germes de céréales; des esters d'acide lanolique, d'acide oléique, d'acide laurique, d'acide stéarique; des alcools tels que l'alcool oléique, l'alcool linoléique ou linolénique, l'alcool isostéarique ou l'octyl dodécanol. On peut également citer les huiles siliconées telles que les polydiméthylsiloxanes, éventuellement phénylées comme par exemple les phényltriméthicones.

Les compositions selon l'invention peuvent également comprendre des huiles volatiles, telles que la cyclotétradiméthylsiloxane, la cyclopentadiméthylsiloxane, la cyclohexadiméthylsiloxane, le méthylhexyldiméthylsiloxane, l'hexaméthyldisiloxane ou les isoparaffines.

De préférence, la composition selon l'invention comprend au moins une huile choisie parmi les isoparaffines volatiles telles que l'isododécane, les huiles non volatiles carbonées telles que le parléam ou le benzoate d'alcool, les polydiméthylsiloxanes, les silicones volatiles et les huiles siliconées phénylées.

Les compositions selon l'invention peuvent en outre comprendre une ou plusieurs matières colorantes additionnelles, pouvant être choisies parmi les colorants hydrosolubles, les colorants liposolubles, et les matières colorantes pulvérulentes comme les pigments, les nacres, et les paillettes bien connues de l'homme du métier.

Ces matières colorantes additionnelles peuvent être présentes dans la composition, en une teneur allant de 0,01 à 50 % en poids, par rapport au poids de la composition, de préférence de 0,02 à 25 % en poids.

Par pigments additionnels, il faut comprendre des particules de toute forme, blanches ou colorées, minérales ou organiques, insolubles dans le milieu physiologique, destinées à colorer la composition. Comme pigment minéral utilisable à titre de pigment additionnel, on peut notamment citer le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, ou la poudre de cuivre.

Comme pigment organique utilisable à titre de pigment additionnel, on peut notamment citer le noir de carbone, les pigments de type D&C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Comme colorant hydrosoluble, on peut notamment citer le sel disodique de ponceau, le sel disodique du vert d'alizarine, le sel trisodique d'amarante, le sel disodique de tartrazine, le sel monosodique de rhodamine, le sel disodique de fuchsine, la xanthophylle, le bleu de méthylène.

Les compositions selon l'invention peuvent comprendre en outre une ou plusieurs charges, notamment en une teneur allant de 0,01 % à 50 % en poids, par rapport au poids total de la composition, de préférence allant de 0,02 % à 30 % en poids.

Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition. Les charges peuvent être minérales ou organiques de toute forme, plaquettaires, sphériques ou oblongues, quelle que soit la forme cristallographique (par exemple feuillet, cubique, hexagonale, orthorhombique, etc.).

Comme exemple de charge, on peut notamment citer le talc, le mica, la silice, le kaolin, les poudres de polyamide (Nylon^{®}) (Orgasol^{®} de chez Atochem), de polo-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de polymères d'acide acrylique (Polytrap^{®} de la société Dow Corning) et les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads^{®} de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les compositions selon l'invention peuvent également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les antioxydants, les agents anti-chutes des cheveux, les agents anti-pelliculaires, les agents propulseurs, les céramides, des actifs cosmétiques, des hydratants, des vitamines, des acides gras essentiels, des polymères, des stabilisants, des colloïdes ou leurs mélanges.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention peuvent se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple.

La composition peut être anhydre, par exemple il peut s'agir d'une pâte anhydre.

Par « anhydre », on entend au sens de la présente invention, une composition comprenant moins de 5 % en poids d'eau, et notamment moins de 3 % en poids d'eau, voire qui est exempte d'eau.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions selon l'invention peuvent être des compositions de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).

Les compositions selon l'invention peuvent être des compositions de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel.

Les compositions selon l'invention peuvent être également des produits capillaires, notamment pour le soin, l'hygiène, le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.

Les compositions selon l'invention peuvent également être des compositions de coloration des cheveux.

Avantageusement, les compositions selon l'invention peuvent être des compositions de maquillage, notamment des ongles ou des lèvres. En particulier, elles peuvent constituer un vernis à ongles ou un rouge à lèvres.

L'invention a aussi pour objet un procédé de traitement cosmétique des matières kératiniques, notamment de la peau du corps ou du visage, des ongles, des cheveux et/ou des cils, comprenant l'application sur lesdites matières d'une composition cosmétique telle que définie précédemment.

Les exemples et figure présentés ci-après sont présentés à titre illustratif et non limitatif du domaine de l'invention.

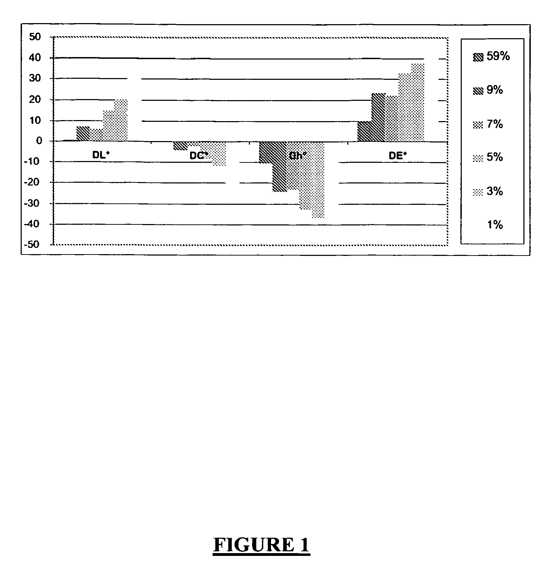

Figure 1 : Ecarts colorimétriques entre le DC Red 7 non encapsulé et le DC Red 7 encapsulé, à différents taux de pigment.

### Exemple 1 : Synthèse de micro capsules comprenant 57,5 % en poids de D&C Red n°7 par rapport au poids de la matrice polymérique

On réalise une émulsion huile-dans-eau comprenant une phase organique constituée de 24 g de diphénylméthane-4,4' diisocyanate (ou MDI) dans laquelle sont dispersés 20 g de D&C Red n°7 (LCW) à encapsuler, et 45 g d'isododécane, et une phase continue aqueuse constituée de 800 g d'eau dans laquelle sont solubilisés 8 g d'éthylène glycol, 2,8 g de triméthylolpropane et 1,7 g de dodécylsulfate de sodium (ou SDS).

Un colloïde protecteur de type alcool polyvinylique PVA hydrolysé à 98 % (Mw=13000-23000) peut être ajouté à 5 % en poids par rapport à la phase aqueuse selon les tailles de particules souhaitées.

L'émulsification est réalisée à fort cisaillement à l'aide d'un Ultraturrax^{®} (entre 6000 et 27000 tr/min) ou d'un sonicateur (entre 10 % et 90 % d'amplitude).

La polymérisation est conduite à 63 °C en présence ou absence de 2 éthylhexanoate d'étain (catalyseur) pendant 4 heures puis à température ambiante durant 15 heures.

En fin de réaction la poudre est récupérée par filtration ou par centrifugation.

### Exemple 2 : Synthèse de micro capsules comprenant 1 % en poids de D&C Red n°7 par rapport au poids de la matrice polymérique

On réalise une émulsion huile-dans-eau comprenant une phase organique constituée de 12g de diphénylméthane-4,4' diisocyanate dans laquelle sont dispersés 0,18 g de D&C Red n°7 et 2,8 g d'isododécane et une phase continue aqueuse constituée de 400 g d'eau dans laquelle est solubilisé 5 % de PVA hydrolysé à 98 % (Mw=13000-23000) et 0,84 g de pluronic F68^{®}. Après 40 min d'émulsification à l'ultraturrax (13500tr/min), on introduit 4g de diéthylène glycol et 1,38 g de triméthylolpropane.

La polymérisation est conduite à 63 °C pendant 4 heures puis à température ambiante durant 15 heures.

En fin de réaction la poudre est récupérée par centrifugation (4000 tr/min pendant 30min).

Les microcapsules obtenues sont caractérisées comme étant de type réservoir par observation au microscope électronique à balayage.

### Exemple 3 : Synthèse de micro capsules comprenant 3 % en poids de D&C Red n°7 par rapport au poids de la matrice polymérique

Le protocole de synthèse est identique à celui décrit en Exemple 2, en utilisant 0,52 g de D&C Red n°7 (au lieu de 0,18 g).

### Exemple 4 : Synthèse de micro capsules comprenant 7 % en poids de D&C Red n°7 par rapport au poids de la matrice polymérique

Le protocole de synthèse est identique à celui décrit en Exemple 2, en utilisant 1,22 g de D&C Red n°7 (au lieu de 0,18 g).

### Exemple 5 : Synthèse de microcapsules comprenant 57 % en poids de D&C Red n°7 par rapport au poids de la matrice polymérique

On réalise une émulsion huile-dans-eau comprenant une phase organique constituée de 24 g de diphenylméthane-4,4' diisocyanate dans laquelle sont dispersés 20 g de D&C Red n°7 et 245,4 g de parléam^{®} et une phase continue aqueuse constituée de 800 g d'eau dans laquelle sont solubilisés 5 % en poids de PVA hydrolysé à 98 % (Mw=13000-23000). Après 40 min d'émulsification à l'Ultraturrax^{®} (13500 tr/min), on introduit 8 g de diéthylène glycol et 2,76 g de triméthylolpropane.

La polymérisation est conduite à 63 °C pendant 4 heures puis à température ambiante durant 15 heures.

En fin de réaction la poudre est récupérée par centrifugation (4000 tr/min pendant 30 min).

Les microcapsules obtenues sont caractérisés comme étant de type réservoir par observation au microscope électronique à balayage.

### Exemple 6 : Synthèse de micro capsules comprenant 30 % en poids de Yellow n°6 aluminium lake par rapport au poids de la matrice polymérique

Le protocole de synthèse est identique à celui décrit en Exemple 2, en utilisant 7,87 de Yellow n°6 aluminium lake (Sun Chemical) (au lieu de 0,18 g de D&C Red n°7) et 2,4g de benzoate d'alcool de sorte à atteindre un rapport massique MDI/benzoate d'alcool = 5).

Les microcapsules obtenues sont caractérisées comme étant de type réservoir par observation au microscope électronique à balayage.

### Exemple 7 : Synthèse de micro capsules comprenant 30 % en poids de Yellow n°6 aluminium lake par rapport au poids de la matrice polymérique

Le protocole de synthèse est identique à celui décrit en Exemple 2, en utilisant 7,87 de Yellow n°6 aluminium lake (Sun Chemical) (au lieu de 0,18 g de D&C Red n°7) et 12 g de benzoate d'alcool (de sorte à atteindre un rapport massique MDI/benzoate d'alcool = 1).

Les microcapsules obtenues sont caractérisés comme étant de type matriciel par observation au microscope électronique à balayage.

### Exemple 8 : Modulation de la teinte d'un pigment en fonction du taux pondéral de pigment encapsulé

L'encapsulation du pigment D&C Red n°7 dans une matrice polymérique de type polyuréthane et polyurée, réalisée en utilisant différents taux pondéraux de pigments tels que précisés dans le Tableau 1 ci-dessous, permet d'accéder à des teintes différentes de celle du pigment initial, et variant selon le taux de pigment encapsulé.

La mesure de la teinte est effectuée sur 1,25 g de poudre compactée à 100 bars dans une coupelle de dimension h x L x 1 =0,3 x 2,5 x 2,2 et recouverte d'une lame de verre.

Les données colorimétriques sont obtenues en réflexion sur un spectrocolorimètre Minolta (D65/10° composante spéculaire exclue petite ouverture).

Le Tableau 1 ci-après rend compte des coordonnées colorimétriques du D&C Red n°7 selon son taux d'encapsulation dans des microcapsules de type polyuréthane et polyurée.

**TABLEAU 1**

| **Taux de pigments** | **L*** | **a*** | **b*** | **c*** | **h°** |
|---|---|---|---|---|---|
| 100 % | 30,51 | 41,38 | 32,48 | 54,58 | 40,6 |
| 1 % | 62,00 | 32,28 | 0,42 | 32,28 | 0,8 |
| 3 % | 50,34 | 42,79 | 2,97 | 42,89 | 4,0 |
| 5 % | 45,19 | 45,96 | 6,73 | 46,45 | 8,3 |
| 7 % | 36,47 | 50,12 | 16,07 | 52,64 | 17,8 |
| 9 % | 37,58 | 48,79 | 14,53 | 50,90 | 16,6 |
| 59 % | 31,01 | 47,17 | 27,66 | 54,68 | 30,4 |

La figure 1 rend compte d'une illustration graphique des valeurs obtenues.

Les résultats démontrent que l'encapsulation permet d'élargir significativement la gamme colorimétrique par rapport au pigment d'origine, c'est-à-dire sous forme non encapsulée.

### Exemple 9: Comparaison du dégorgement du D&C Red n°7 pur et du D&C Red n°7 encapsulé à 5 %

Le D&C Red n°7 sous une forme encapsulée ou non (témoin) est placé dans une eau contenant 1 % de polysorbate (1686 M), puis la solution est ensuite centrifugée à 3000 tr/min pendant 30 min.

On détermine ensuite pour chacune des deux solutions la valeur d'absorbance du dégorgeat par mesure de leur densité optique à une valeur de 535 nm. Si nécessaire, le dégorgeat est dilué jusqu'à obtenir une valeur d'absorbance comprise entre 0 et 1.

Le dégorgement correspond à la valeur de la densité optique.

Le Tableau 2 ci-après rend compte des valeurs obtenues.

**TABLEAU 2**

| Facteur de dilution | Par dilution | 1/16 |
|---|---|---|
| DCRed 7 pur | 10,000 | 0,401 |
| DCred7 encapsulé à 5 % | 0,226 | |

On constate que l'encapsulation réduit considérablement le dégorgement.

### Exemple 10 : Formulation de rouge à lèvres

| | % massique |
|---|---|
| Ditertiobutyl 4-hydroxytoluène | qs |
| Cire microcristalline | 4 |
| Tri-mellitate de tri-décyle | 10 |
| Lanoline liquide | 9 |
| Malate de di-iso-stéaryle | 13 |
| Lanoline acétylée protégée | 9 |
| Triglycérides d'acides laurique/palmitique/acétylique/stéarique (50/20/10/10) | 5 |
| Lanolate d'isopropyle protégée | 9 |
| Octyl-2 dodécanol | Qsp 100 |
| D&C Red n°7 encapsule à 57% (Exemple 5) | 10 |
| Phényl triméthylsiloxy trisiloxane (viscosité : 20 CST - PM: 372) | 4 |
| Cire de polyéthylène (PM: 500) | 8 |

## Revendications

1. Composition cosmétique et/ou dermatologique pour le maquillage et/ou le soin d'une matière kératinique, **caractérisée en ce qu'**elle comprend un milieu physiologiquement acceptable, ainsi que :
au moins un matériau colorant composite se présentant sous la forme de microcapsules composées :
(a) d'une matrice polymérique de type polyuréthane, polyurée et/ou polyuréthane et polyurée réticulée obtenue par polycondensation interfaciale en faisant réagir
au moins un diisocyanate choisi parmi les mélanges de monomères diphénylméthane diisocyanates et d'oligomères diphénylméthane diisocyanates, le 2,4-tolylène diisocyanate et le 2,6-tolylène diisocyanate, ainsi que leur mélange, le 4,4'-diphénylméthane diisocyanate ou l'isophorone diisocyanate ;
avec au moins un réactif de type alcool et/ou amine portant au moins trois fonctions, identiques ou différentes, choisies parmi les fonctions hydroxyle et amine, en présence éventuellement d'au moins un diol et/ou d'au moins une diamine et/ou d'au moins un amino-alcool difonctionnel, et
(b) d'un pigment, lesdites microcapsules comprenant de 10 à 80 % en poids du pigment par rapport au poids de la matrice polymérique.

2. Composition selon la revendication 1, **caractérisée en ce qu'**elle comprend de 0,01 % à 50 % en poids, notamment de 0,1 à 20 % en poids, et en particulier de 0,5 à 15 % en poids, par rapport à son poids total, de matériau colorant composite.

3. Composition selon la revendication 1 ou 2, **caractérisée en ce que** le pigment est au moins en partie organique.

4. Composition selon la revendication 3, **caractérisée en ce que** le pigment est un pigment organique.

5. Composition selon l'une quelconque des revendications précédentes, **caractérisée en ce que** les microcapsules comprennent de 15 à 75 % en poids, notamment de 20 à 70 % en poids, voire de 25 à 65 % en poids et de préférence de 30 à 60 % en poids du pigment par rapport au poids de la matrice polymérique.

6. Composition selon l'une quelconque des revendications précédentes, **caractérisée en ce que** les microcapsules sont de type réservoir.

7. Composition selon la revendication 6, **caractérisée en ce que** le pigment est emprisonné dans l'écorce des microcapsules.

8. Composition selon la revendication 6, **caractérisée en ce que** le pigment est emprisonné dans le coeur des microcapsules.

9. Composition selon l'une quelconque des revendications 1 à 5, **caractérisée en ce que** les microcapsules sont de type matriciel.

10. Composition selon l'une quelconque des revendications précédentes, **caractérisée en ce que** la matrice polymérique desdites microcapsules est de type polyuréthane et polyurée.

11. Composition selon la revendication 1, dans laquelle le réactif alcool portant au moins trois fonctions hydroxyle est choisi parmi les triols, comme par exemple le triméthylolpropane, le glycérol ou le pentaéryhtrol, et les polyols, comme par exemple les sucres tels le glucose, le fructose, le xylose, le tréhalose, le saccharose, le maltose et le lactose.

12. Composition selon l'une quelconque des revendications précédentes, dans laquelle le réactif de type amine comprend au moins un groupe amino choisi parmi les groupes amine primaires et amine secondaires de type -NHR, dans lequel R représente un groupe alkyle ayant de 1 à 8 atomes de carbone.

13. Composition selon l'une quelconque des revendications précédentes, dans laquelle le réactif de type amino-alcool est la triéthanol amine.

14. Composition selon l'une quelconque des revendications précédentes, dans laquelle le pigment est choisi parmi l'oxyde de titane, l'oxyde de chrome, le D&C Red n°7, le D&C Red n°22 lake, le Yellow n°6 lake, et le FD&C Blue n°1 lake.

15. Composition selon l'une quelconque des revendications précédentes, **caractérisée en ce que** le matériau composite comprend en outre au moins une substance non colorante choisie parmi les huiles et les cires cosmétiques, notamment minérales, animales, végétales ou de synthèse, les actifs cosmétiques tels que les vitamines, les filtres UV, les parfums et les hydratants.

16. Composition pour la coloration des cheveux **caractérisée en ce qu'**elle comprend une composition telle que définie selon l'une quelconque des revendications précédentes.

17. Composition de maquillage, notamment des ongles ou des lèvres, **caractérisée en ce qu'**elle comprend une composition telle que définie selon l'une quelconque des revendications précédentes.

18. Procédé de traitement cosmétique des matières kératiniques, notamment de la peau du corps ou du visage, des ongles, des cheveux et/ou des cils, comprenant l'application sur lesdites matières d'une composition cosmétique telle que définie dans l'une quelconque des revendications 1 à 17.

## Patentansprüche

1. Kosmetische und/oder dermatologische Zusammensetzung zum Schminken und/oder zur Pflege einer keratinischen Substanz, **dadurch gekennzeichnet, dass** sie ein physiologisch verträgliches Medium, sowie folgendes umfasst:
mindestens einen Verbundfarbstoff, der in Form von Mikrokapseln vorliegt, die bestehen aus:
(a) einer Polymermatrix vom Typ Polyurethan, Polyharnstoff und/oder Polyurethan und vernetzter Polyharnstoff, erhalten durch Grenzflächen-Polykondensation unter Umsetzung von
mindestens einem Diisocyanat, das ausgewählt ist aus den Gemischen von Diphenylmethandiisocyanat-Monomeren und Diphenylmethandiisocyanat-Oligomeren, 2,4-Toluylendiisocyanat und 2,6-Toluylendiisocyanat, sowie ihren Gemischen, 4,4'-Diphenylmethandiisocyanat oder Isophorondiisocyanat;
mit mindestens einem Reagenz von Typ Alkohol und/oder Amin, das mindestens drei gleiche oder verschiedene Funktionen trägt, die ausgewählt sind aus Hydroxyl- und Aminfunktionen, in Gegenwart von gegebenenfalls mindestens einem Diol und/oder mindestens einem Diamin und/oder mindestens einem bifunktionellen Alkohol, und
(b) einem Pigment, wobei die Mikrokapseln 10 bis 80 Gew.-% des Pigments bezogen auf das Gewicht der Polymermatrix umfassen.

2. Zusammensetzung nach Anspruch 1, **dadurch gekennzeichnet, dass** sie 0,01 bis 50 Gew.-%, insbesondere 0,1 bis 20 Gew.-% und insbesondere 0,5 bi 15 Gew.-% bezogen auf das Gesamtgewicht an Verbundfarbstoff umfasst.

3. Zusammensetzung nach Anspruch 1 oder 2, **dadurch gekennzeichnet, dass** das Pigment mindestens zum Teil organisch ist.

4. Zusammensetzung nach Anspruch 3, **dadurch gekennzeichnet, dass** das Pigment ein organisches Pigment ist.

5. Zusammensetzung nach einem der vorhergehenden Ansprüche, **dadurch gekennzeichnet, dass** die Mikrokapseln 15 bis 75 Gew.-%, insbesondere 20 bis 70 Gew.-%, auch 25 bis 65 Gew.-% und vorzugsweise 30 bis 60 Gew.-% Pigment bezogen auf das Gewicht der Polymermatrix umfassen.

6. Zusammensetzung nach einem der vorhergehenden Ansprüche, **dadurch gekennzeichnet, dass** die Mikrokapseln von der Art eines Reservoirs sind.

7. Zusammensetzung nach Anspruch 6, **dadurch gekennzeichnet, dass** das Pigment in der Hülle der Mikrokapseln eingeschlossen ist.

8. Zusammensetzung nach Anspruch 6, **dadurch gekennzeichnet, dass** das Pigment im Kern der Mikrokapseln eingeschlossen ist.

9. Zusammensetzung nach einem der Ansprüche 1 bis 5, **dadurch gekennzeichnet, dass** die Mikrokapseln matrixartig sind.

10. Zusammensetzung nach einem der vorhergehenden Ansprüche, **dadurch gekennzeichnet, dass** die Polymermatrix der Mikrokapseln vom Typ Polyurethan und Polyharnstoff ist.

11. Zusammensetzung nach Anspruch 1, wobei das Alkohol-Reagens, das mindestens drei Hydroxylfunktionen trägt, ausgewählt ist aus Triolen, wie beispielsweise Trimethylolpropan, Glycerin oder Pentaerythrit, und Polyolen, wie beispielsweise Zuckern, wie Glucose, Fruktose, Xylose, Trehalose, Saccharose, Maltose und Laktose.

12. Zusammensetzung nach einem der vorhergehenden Ansprüche, wobei das Reagens von der Art eines Amins mindestens eine Aminogruppe umfasst, die ausgewählt ist primären und sekundären Amingruppen des Typs -NHR, wobei R eine Alkylgruppe mit 1 bis 8 Kohlenstoffatomen darstellt.

13. Zusammensetzung nach einem der vorhergehenden Ansprüche, wobei das Reagens von der Art eines Aminoalkohols Triethanolamin ist.

14. Zusammensetzung nach einem der vorhergehenden Ansprüche, wobei das Pigment ausgewählt ist aus Titanoxid, Chromoxid, D&C Red No. 7, D&C Red No. 22 lake, Yellow No. 6 lake and FD&C Blue No. 1 lake.

15. Zusammensetzung nach einem der vorhergehenden Ansprüche, **dadurch gekennzeichnet, dass** das Verbundmaterial weiterhin mindestens eine Nichtfarbstoffsubstanz umfasst, die ausgewählt ist aus kosmetischen Ölen und Wachsen, insbesondere mineralischen, pflanzlichen oder synthetischen, kosmetischen Wirkstoffen, wie Vitaminen, UV-Filtern, Duftstoffen und Feuchtigkeitsspendern.

16. Zusammensetzung zur Färbung der Haare, **dadurch gekennzeichnet, dass** sie eine Zusammensetzung nach einem der vorhergehenden Ansprüche umfasst.

17. Zusammensetzung zum Schminken, insbesondere der Nägel oder der Lippen, **dadurch gekennzeichnet, dass** sie eine Zusammensetzung nach einem der vorhergehenden Ansprüche umfasst.

18. Verfahren zur kosmetischen Behandlung von keratinischen Substanzen, insbesondere der Haut des Körpers oder des Gesichts, der Nägel, der Haare und/oder der Wimpern, umfassend das Auftragen auf die Substanzen einer kosmetischen Zusammensetzung nach einem der Ansprüche 1 bis 17.

## Claims

1. A cosmetic and/or dermatological composition for making up and/or caring for a keratinous material, **characterized in that** it comprises a physiologically acceptable medium, as well as:
at least one composite coloring material appearing as microcapsules consisting of:
(a) a cross-linked polymeric matrix of the polyurethane, polyurea and/or polyurethane and polyurea type obtained by interfacial polycondensation by reacting
at least one diisocyanate selected from among mixtures of diphenymethane diisocyanates monomers and of diphenylmethane diisocyanate oligomers, 2,4-tolylene diisocyanate and 2,6-tolylene diisocyanate, as well as a mixture thereof, 4,4'-diphenylmethane diisocyanate or isophorone diisocyanate;
with at least one reagent of the alcohol and/or amine type bearing at least three functions, either identical or different, selected from hydroxyl or amine functions, optionally in the presence of at least one diol and/or of at least one diamine and/or of at least one difunctional amino-alcohol, and
(b) of a pigment, said microcapsules comprising from 10 to 80% by weight of the pigment based on the weight of the polymeric matrix.

2. The composition according to claim 1, **characterized in that** it comprises from 0.01% to 50% by weight, notably from 0.1 to 20% by weight, and in particular from 0.5 to 50% by weight based on its total weight, of composite coloring material.

3. The composition according to claim 1 or 2, **characterized in that** the pigment is at least partly organic.

4. The composition according to claim 3, **characterized in that** the pigment is an organic pigment.

5. The composition according to any of the preceding claims, **characterized in that** the microcapsules comprise from 15 to 75% by weight, notably from 20 to 70% by weight, or even from 25 to 65% by weight and preferably from 30 to 60% by weight of the pigment based on the weight of the polymeric matrix.

6. The composition according to any of the preceding claims, **characterized in that** the microcapsules are of the reservoir type.

7. The composition according to claim 6, **characterized in that** the pigment is confined in the shell of the microcapsules.

8. The composition according to claim 6, **characterized in that** the pigment is confined in the core of the microcapsules.

9. The composition according to any of claims 1 to 5, characterizing that the microcapsules are of the matrix type.

10. The composition according to any of the preceding claims, **characterized in that** the polymeric matrix of said microcapsules is of the polyurethane and polyurea type.

11. The composition according to claim 1, wherein the alcohol reagent bearing at least three hydroxyl functions is selected from triols, such as for example trimethylolpropane, glycerol or pentaerythritol, and polyols such as for example sugars like glucose, fructose, xylose, trehalose, saccharose, maltose and lactose.

12. The composition according to any of the preceding claims, wherein the reagent of the amine type comprises at least one amino group selected from primary amine and secondary amine groups of the -NHR type, wherein R represents an alkyl group having from 1 to 8 carbon atoms.

13. The composition according to any of the preceding claims, wherein the reagent of the amino-alcohol type is triethanolamine.

14. The composition according to any of the preceding claims, wherein the pigment is selected from titanium oxide, chromium oxide, D&C Red no. 7, D&C Red no. 22 lake, Yellow no.6 lake, and FD&C Blue no.1 lake.

15. The composition according to any of the preceding claims, **characterized in that** the composite material further comprises at least one non-coloring substance selected from cosmetic, notably mineral, animal, plant or synthetic waxes and oils, cosmetic actives such as vitamins, UV filters, perfumes and moisturizers.

16. A composition for coloring hair, **characterized in that** it comprises a composition as defined according to any of the preceding claims.

17. A composition for making up, notably nails or lips, **characterized in that** it comprises a composition as defined according to any of the preceding claims.

18. A method for cosmetic treatment of keratinous materials, notably of the skin of the body or of the face, of the nails, hair and/or eyelashes, comprising the application on said materials of a cosmetic composition as defined in any of claims 1 to 17.
